# EUROPEAN PATENT SPECIFICATION

(11) **EP 3 446 658 B1**
(45) Date of publication and mention of the grant of the patent: **01.10.2025**
(21) Application number: 17786236.4
(22) Date of filing: 17.04.2017
(51) Int. Cl.: A61F 2/14

(54) **ARTIFICIAL IRIS**
KÜNSTLICHE IRIS
IRIS ARTIFICIEL

(30) Priority: 22.04.2016 RU 2016115972
(43) Date of publication of application: 27.02.2019
(73) Proprietor: Limited Liability Company Enterprise"Reper-NN", 603003, Nizhny Novgorod (RU)
(72) Inventor: SOBOLEV, Nikolay Petrovich, Moscow 123181 (RU); ZIMITSKAYA, Anastasiya Yurevna, Nizhny Novgorod 603076 (RU); JONES, Mihail Mihaylovich, Nizhny Novgorod 603018 (RU)
(74) Representative: Osmans, Voldemars
(86) International application number: PCT/RU2017/000241
(87) International publication number: WO 2017/184031

(56) References cited:
- EP-A1- 1 674 049
- WO-A1-02/47584
- CN-B- 102 090 941
- RU-C1- 2 526 245
- RU-C2- 2 275 174
- US-A1- 2011 264 210
- POZDEYEVA N A ET AL: "Artificial iris-lens diaphragm in reconstructive surgery for aniridia and aphakia", JOURNAL CATARACT AND REFRACTIVE SURGERY, SURGERY, FAIRFAX, VA, US, vol. 31, no. 9, 1 September 2005 (2005-09-01), pages 1750 - 1759, XP027831395, ISSN: 0886-3350, [retrieved on 20050901]

## Description

### FIELD OF THE INVENTION

The invention relates to the field of medicine, more particularly to ophthalmology, and more particularly to reconstructive surgery for pathology of the iris pathology or combined pathology of the iris and lens.

### BACKGROUND OF THE INVENTION

Combination of eye-lens opacity with severe iris defects up to total aniridia may be the result of congenital pathology or serious eye injury. Impairment or lack of iris diaphragmatic function leads not only to vision acuity reduction, but also to the formation of a serious cosmetic defect, that can interfere with a social adaptation and professional activity of a person. Different models of artificial iris combined with artificial eye-lens or without it are used in the clinical practice. They are implanted into the eye cavity through a small incision using an injector.

Artificial iris or iris-lens diaphragm and its' production method are known, patent is RU 2526245, published on 20.08.2014, A61F9/007, A61F2/14. This artificial iris consists of optics and haptics, which are one-piece and made of flexible material. Haptics includes a colored ring and support elements, located on the edge of the colored ring. The colored ring has a pattern imitating a netlike radial pattern of a person's fellow eye iris by its form and color. Support elements are arc-shaped open-ended for one-point contact that are capable of bending in the plane of the colored ring. This iris-lens diaphragm according patent RU 2526245 is the closest analogue to the invention.

Experience has proven that in some surgery cases related to the implantation of iris-lens diaphragm according patent RU 2526245 in post-operative period support elements bended and moved iris-lens diaphragm out-of-plane due to their insufficient rigidity. Iris-lens diaphragm production method, according to patent RU 2526245, allows to increase rigidity of support elements only by enlargement of the total haptics thickness or by making the iris-lens diaphragm of more tough material. But it is unacceptable as it leads to impossibility of iris-lens diaphragm implantation through the small incision using the injector. Disadvantage of the closest analogue is insufficient rigidity of supporting elements for secure retention of the iris-lens diaphragm in the post-operative period.

### BRIEF SUMMARY OF THE INVENTION

The technical result is aimed at providing support elements with the necessary rigidity and suitable for secure retention, keeping the implantation of an artificial iris easy.

The technical result is achieved in that the proposed artificial iris is made of an elastic material in the form of a colored ring with peripheral support elements capable of bending in the plane of the colored ring, wherein the support elements are closed and their thickness thereof exceeds the thickness of the colored ring.

The invention is set out in the appended set of claims.

Material resilience of support elements may exceed material resilience of the colored ring. Colored ring may include a flange on the edge. Thickness of the colored ring varies from 0.1 to 0.4 mm, and thickness of the support elements is not more than 0.6 mm. The colored ring may include optics depending on clinical case.

### BRIEF DESCRIPTION OF SEVERAL VIEWS OF THE DRAWINGS

The invention is depicted in the following drawings.

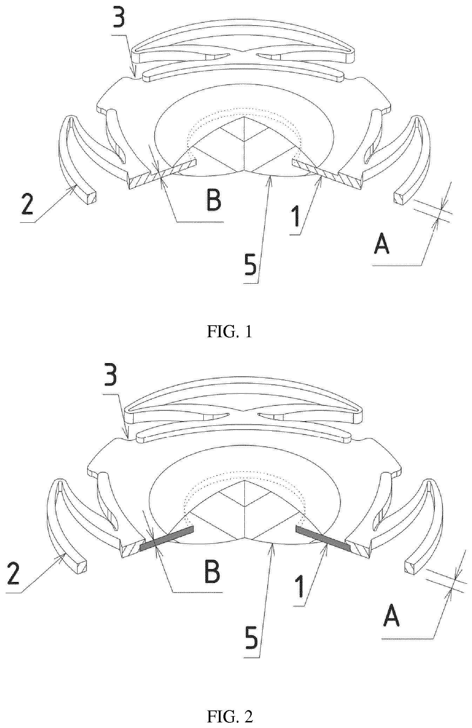

FIGS. 1-4 depict sectional views of exemplary embodiment of an artificial iris.

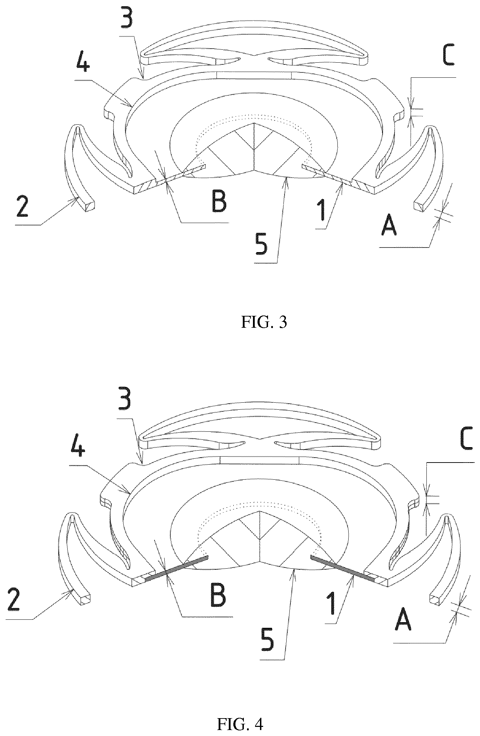

FIGS. 1-4 depict view of exemplary embodiment of an artificial iris that is made in the form of a colored ring **1** comprising peripheral closed support elements **2.** The colored ring **1** has a pattern imitating the person's fellow eye iris by its form and color. Support elements **2** can be bent in a plane of the colored ring **1.** At the base of the support element **2,** slots **3** are formed, into which the support element inserts partially or fully while bending. Artificial iris can include a flange **4** on the edge of the colored ring **1** as shown in FIGS.3 and 4. Flange **4** can be made of the same material as the support elements **2** like in the FIG. 3, or of another material as shown in the FIG.4.

Thickness **C** of the flange **4** mainly coincides with the thickness **A** of the support elements **2.** Thickness **A** of the support elements **2** exceeds the thickness **B** of the colored ring **1,** where **A** is not more 0.6 mm, **B** - 0.1-0.4 mm. The colored ring **1** may include optics **5** depending on clinical case.

### EMBODIMENT OF INVENTION

The colored ring **1** and support elements **2** can be made of material with similar (FIG.1) or different resilience (FIG.2). In case the colored ring **1** and support elements **2** have different resilience the resilience of support elements **2** exceeds resilience of the colored ring **1.** Artificial iris by the invention can be manufactured by photo polymerization of the material consisting of methacrylates with different structure: octyl methacrylate, methacrylate with linear hydrocarbon side chain, methacrylate with branched hydrocarbon side chain. Material In case the colored ring **1** and support elements **2** have different resilience of artificial iris parts varies due to different proportion of the components mentioned above. However, the given example of production method does not limit all possible production methods, which can be different.

Performance of the invention is described below.

Artificial iris is placed in the ciliary sulcus. Support elements **2** bump against ciliary sulcus and are bent in a plane of the colored ring **1,** then fall in line with an individual diameter of the ciliary sulcus, which usually varies from 11.0 to 12.5 mm. Support elements **2** make artificial iris self-centering. Proposed proportion of material thickness and flexibility of support elements **2** and colored ring **1** provides secure artificial iris positioning in the ciliary sulcus in post-operative period. In addition, due to thinner and more flexible colored ring **1** can be easily rolled up to sizes sufficient for injector implantation through the small incision and then it straightens slowly to flat form when leaving the injector. In such a way, risk of surgical eye injury is reduced. Flange **4** mitigates load on the thin colored ring **1** from support elements **2** and allows to prevent uncontrolled bends of the artificial iris in post-operative period.

Implantation of the proposed invention is done as follows.

Artificial iris is intended for injector implantation. Tunnel corneal incision of 2.2 mm wide is made. Artificial iris is put on the glide spatula, narrow gap of which is placed in the cartridge entry. Artificial iris is put into viscoelastic. It is moved inside the cartridge to the narrow part using the elastic pusher, then this function is performed by viscoelastic, located between pusher and artificial iris. After iris placement in the eye cavity using the Sinskey hook for rotation the support elements 2 are adjusted sequentially in the ciliary sulcus. If necessary, the support elements 2 are fixed with a 9.0 polypropylene suture intrascleraly: five limbus parallel sequential and knotless stitches are made at the distance of 1.5-3 mm from the limbus.

Proposed artificial iris provides required rigidity and security of supporting elements fixation keeping the ease of artificial iris implantation.

## Claims

1. Artificial iris made of elastic material for injector implantation comprising a colored ring (1) and peripheral support elements (2) capable of bending in the plane of the colored ring (1), wherein slots (3) are formed at a base of the support elements (2), **characterized in that**
the support elements (2) are in a form of a closed loop, a thickness (A) of the support elements (2) exceeds a thickness (B) of the colored ring (1), and the slots (3) accommodate the loops when bending.

2. Artificial iris of claim 1, wherein a material of the colored ring (1) and a material of the support elements (2) have a different resilience, the resilience of the support elements (2) exceeding the resilience of the colored ring (1).

3. Artificial iris of claim 1, further comprising a flange (4) shaped as continuous peripheral ring on an edge of the colored ring (2) and forming the base of the support elements (2).

4. Artificial iris of claim 1, wherein the thickness (B) of the colored ring (1) varies from 0.1 mm to 0.4 mm.

5. Artificial iris of claim 1, wherein the thickness (A) of the support elements (2) is not more than 0.6 mm.

6. Artificial iris of claim 1, wherein the colored ring (1) includes optics.

## Patentansprüche

1. Künstliche Iris aus elastischem Material zur Injektorimplantation, bestehend aus einem farbigen Ring (1) und peripheren Stützelementen (2), die sich in der Ebene des farbigen Rings (1) biegen können, wobei an einer Basis der Stützelemente (2) Schlitze (3) ausgebildet sind, **dadurch gekennzeichnet, dass** die Stützelemente (2) die Form einer geschlossenen Schleife haben, Eine Dicke (A) der Stützelemente (2) übersteigt eine Dicke (B) des farbigen Rings (1), und die Schlitze (3) nehmen die Schlaufen beim Biegen auf.

2. Künstliche Iris nach Anspruch 1, wobei ein Material des farbigen Rings (1) und ein Material der Stützelemente (2) eine unterschiedliche Elastizität aufweisen, wobei die Elastizität der Stützelemente (2) die Elastizität des farbigen Rings (1) übersteigt.

3. Künstliche Iris nach Anspruch 1, die außerdem einen Flansch (4) umfasst, der als durchgehender Umfangsring an einer Kante des farbigen Rings (2) geformt ist und die Basis der Stützelemente (2) bildet.

4. Künstliche Iris nach Anspruch 1, wobei die Dicke (B) des farbigen Rings (1) zwischen 0,1 mm und 0,4 mm variiert.

5. Künstliche Iris nach Anspruch 1, wobei die Dicke (A) der Stützelemente (2) nicht mehr als 0,6 mm beträgt.

6. Künstliche Iris nach Anspruch 1, wobei der farbige Ring (1) eine Optik umfasst.

## Revendications

1. Iris artificiel en matériau élastique pour implantation par injecteur comprenant un anneau coloré (1) et des éléments de support périphériques (2) capables de se plier dans le plan de l'anneau coloré (1), **caractérisé en ce que** des fentes (3) sont formées à la base des éléments de support (2), **caractérisé en ce que** les éléments de support (2) sont en forme de boucle fermée, une épaisseur (A) des éléments de support (2) dépasse une épaisseur (B) de l'anneau coloré (1), et les fentes (3) reçoivent les boucles lors de la flexion.

2. Iris artificiel selon la revendication 1, **caractérisé en ce que** un matériau de l'anneau coloré (1) et un matériau des éléments de support (2) ont une résilience différente, la résilience des éléments de support (2) dépassant la résilience de l'anneau coloré (1).

3. Iris artificiel selon la revendication 1, comprenant une bride (4) en forme d'anneau périphérique continu sur un bord de l'anneau coloré (2) et formant la base des éléments de support (2).

4. Iris artificiel selon la revendication 1 **caractérisé en ce que** l'épaisseur (B) de l'anneau coloré (1) varie de 0,1 mm à 0,4 mm.

5. Iris artificiel selon la revendication 1 **caractérisé en ce que** l'épaisseur (A) des éléments de support (2) n'est pas supérieure à 0,6 mm.

6. Iris artificiel selon la revendication 1 **caractérisé en ce que** l'anneau coloré (1) comprend des éléments optiques.
